# EUROPÄISCHE PATENTSCHRIFT

(11) **EP 0 830 874 B1**
(45) Veröffentlichungstag und Bekanntmachung des Hinweises auf die Patenterteilung: **05.12.2001**
(21) Anmeldenummer: 97116044.5
(22) Anmeldetag: 16.09.1997
(51) Int. Cl.: A61M 39/14, A61J 1/05

(54) **Sterile Konnektoranordnung, insbesondere zum medizinischen Gebrauch**
Connector device for medical use
Dispositif de connecteur en particulier pour usage médical

(30) Priorität: 17.09.1996 DE 19637856
(43) Veröffentlichungstag der Anmeldung: 25.03.1998
(73) Patentinhaber: Fresenius AG, 61350 Bad Homburg v.d.H (DE)
(72) Erfinder: Knierbein, Bernd, Dr., 66606 St. Wendel (DE); Lauer, Martin, 66606 St. Wendel (DE)
(74) Vertreter: Luderschmidt, Schüler & Partner GbR

(56) Entgegenhaltungen:
- EP-A- 0 288 250
- CA-A- 2 171 652
- DE-A- 3 244 151
- DE-U- 29 515 682
- US-A- 4 344 472

## Beschreibung

Die Erfindung betrifft eine sterile Konnektoranordnung zum Anschluß einer Schlauchleitung an einen Behälter, der eine medizinische Flüssigkeit enthält sowie einen Behälter mit einer sterilen Konnektoranordnung.

Ein Folienbeutel für medizinische Flüssigkeiten mit einer derartigen Konnektoranordnung ist aus US-A-4,201,406 bekannt. Der bekannte Beutel weist einen schlauchförmigen Anschlußstutzen auf, der mit der Folie verschweißt und mit einer durchstechbaren Membran verschlossen ist, die von einem Einstechdorn, der auch als Spike bezeichnet wird, durchstoßen werden kann. Der Spike wird von einem rohrförmigen Körper konzentrisch umgeben, der sich auf den schlauchförmigen Anschlußstutzen aufschieben läßt, wobei die Membran durchstoßen und eine Fluidverbindung zwischen dem Beutelinnern und der an dem Spike angeschlossenen Schlauchleitung hergestellt wird. US-A-5,125,919 beschreibt einen Folienbeutel für medizinische Flüssigkeiten, dessen Anschlußstutzen ein im wesentlichen kastenförmiges Basisteil aufweist, das mit dem Folienbeutel verschweißt ist.

CA-A₁-2 171 652 offenbare eine Konnektoranordnung laut das erste Teil des 1. Anspruchs.

Mit den oben genannten Konnektoren lassen sich die Behälter zwar steril verschließen, als nachteilig erweist sich jedoch, daß der vor der Membran liegende Teil der kanalförmigen Ausnehmung, in die der Spike eingeführt wird, ungeschützt ist. Somit besteht die Gefahr, daß beim Durchstoßen der Membran Keime in den Behälter eingebracht werden.

Bekannte Verschlußelemente, die eine Verkeimung des Behälterinhalts vermeiden sollen, weisen einen Verschlußkörper aus Weich-PVC mit einer kanalförmigen von einer Membran verschlossenen Ausnehmung auf, der an seinem anschlußseitigen Ende in einer Schweißpresse plattgedrückt und dicht zugeschweißt ist. Oberhalb der Membran ist der Verschlußkörper mit einer ringförmigen Schwächungszone versehen, so daß das Oberteil des Verschlußkörpers von dessen Unterteil abgedreht werden kann. Um die Teile des Verschlußkörpers fassen und ein großes Drehmoment aufbringen zu können, sind an dem Verschlußkörper direkt ober- und unterhalb der Schwächungszone radial nach außen abstehende Flügel vorgesehen. Bei diesen Konnektoren wird die unregelmäßig geformte Schweißzone am oberen Ende des Anschlußstücks im allgemeinen als störend empfunden. Ferner ist nachteilig, daß die Konnektoren nur aus Materialien, z.B. Weich-PVC, hergestellt werden können, die sich zum Verschließen der kanalförmigen Ausnehmung in einer Schweißpresse leicht zusammendrücken und verformen lassen. Darüber hinaus besteht die Gefahr, daß beim Abdrehen des Konnektoroberteils sich an der Sollbruchstelle lösende Partikel in die kanalförmige Ausnehmung fallen. Als nachteilig erweist sich auch, daß der nach dem Abdrehen des Oberteils freiliegende Öffnungsbereich des Verschlußkörpers relativ steif ist. Dies ist darauf zurückzuführen, daß der Verschlußkörper unterhalb der Schwächungszone sehr dickwandig ausgebildet ist. Im übrigen führen auch die radial abstehenden Flügel noch zu einer zusätzlichen Versteifung. Dies ist insofern nachteilig, als eine sichere Abdichtung des in die kanalförmige Ausnehmung einzuführenden Einstechdorns nicht dadurch erreicht werden kann, daß der Dorn unter Aufweitung des Verschlußkörpers in diesen eingeschoben wird. Die kanalförmige Ausnehmung läßt sich nur dann sicher abdichten und der Einstechdorn leicht in den Verschlußkörper einführen, wenn das Material, aus dem der Verschlußkörper besteht, elastisch verformbar ist. Daher können auch aus diesem Grund Materialien mit einem hohen E-Modul nicht Verwendung finden.

Der Erfindung liegt die Aufgabe zugrunde, eine sterile Konnektoranordnung zum Anschluß einer Schlauchleitung an einen eine medizinische Flüssigkeit enthaltenen Behälter zu schaffen, die eine hohe Sicherheit gegen Verkeimen des Behälters bietet, deren Einstechdorn sicher abdichtet und die sich leicht handhaben läßt.

Eine weitere Aufgabe der Erfidnung liegt darin, einen Behälter mit einer sterilen Konnektoranordnung zur Verfügung zu stellen, der eine hohe Sicherheit gegen Verkeimen bietet, dessen Inhalt unter sterilen Bedingungen entnommen werden kann und der sich leicht handhaben läßt.

Die Lösung dieser Aufgabe erfolgt erfindungsgemäß mit den Merkmalen des Patentanspruchs 1 bzw. 11.

Bei der erfindungsgemäßen Konnektoranordnung ist eine Schutzkappe vorgesehen, die den rohrförmigen Abschnitt des in die Behälterwand einsetzbaren Anschlußstücks übergreift. Die Schutzkappe ist mit ihrem unteren Rand mit dem Anschlußstück verbunden und weist im Bereich ihres unteren Randes eine Ringbruchzone auf. Sie dient nicht nur zum bakteriellen Schutz der Einfüllstelle, sondern stellt auch einen Originalitätsverschluß dar.

Vor dem Einführen des Duchstoßelementes in die kanalförmige Ausnehmung des Anschlußstücks wird die Schutzkappe von dem Anschlußstück durch Drehen derselben abgebrochen. Da der rohrförmige Abschnitt des Anschlußstücks erst jetzt freiliegt, besteht eine nur sehr geringe Gefahr, daß beim Einführen des Durchstoßelementes Keime in den Behälter eingebracht werden. Vorteilhaft ist, wenn die Schutzkappe einen relativ kleinen Durchmesser hat und die Wand der Schutzkappe im Bereich der Ringbruchzone relativ dünnwandig ausgebildet ist, so daß sich die Schutzkappe leicht abbrechen läßt.

Bei der Herstellung der Konnektoranordnung kann die Schutzkappe direkt nach dem Spritzgießen im sterilen Zustand mit dem Anschlußstück verschweißt werden. Damit ist der Raum vor der Membran dicht abgeschlossen. Für den Fall, daß die Verschweißung nach dem Spritzgießen unter nicht sterilen Bedingungen durchgeführt wird, kann ein Wassertropfen in den abgeschlossenen Hohlraum eingebracht werden, der während der Autoklavierung verdampft und so die sichere Sterilisation des Hohlraumes erlaubt. Auch eine trockene Heißsterilisation der verschweißten Spritzteile unter höheren Temperaturen und/oder mit längeren Zeiten ist möglich. Ferner kann die Sterilisation mit Gamma-Strahlen erfolgen.

Von Vorteil ist, daß zum dichten Abschluß des rohrförmigen Abschnitts keine elastomeren Bauteile wie Silikon-O-Ringe benötigt werden, die mit größerem Herstell- und Montageaufwand verbunden sind, und daß die Sortenreinheit der gesamten Konnektoranordnung gewährleistet ist. Da die Ringbruchzone außerhalb des Öffnungsbereiches des rohrförmigen Abschnitts des Anschlußstücks liegt, besteht nicht die Gefahr, daß beim Abbrechen der Schutzkappe Partikel in die kanalförmige Ausnehmung des Anschlußstücks fallen.

Um den Einstechdorn leicht in die kanalförmige Ausnehmung des Anschlußstücks einführen und die Membran durchstoßen zu können, ist eine Überwurfmutter vorgesehen, die nach dem Entfernen der Schutzkappe auf den mit einem Außengewinde versehenen ringförmigen Abschnitt des Anschlußstücks aufgeschraubt werden kann. Durch Drehen der auf das Anschlußstück aufgesetzten Überwurfmutter wird der Einstechdorn in axiale Richtung verschoben, wodurch die Membran durchstoßen wird. Während dieses Anstechvorganges wird der Raum vor der Membran durch den Einstechdorn mit der Überwurfmutter abgedeckt. Der Durchmesser der kanalförmigen Ausnehmung des Anschlußstücks und der Durchmesser des Einstechdorns ist vorteilhafterweise derart bemessen, daß der Einstechdorn gegen das Anschlußstück steril abdichtet, bevor die Membran durchstoßen wird. Somit kann die Schlauchleitung auch dann leckfrei an den Behälter angeschlossen werden, wenn die Konnektoranordnung an der Unterseite des Behälters angeordnet ist oder der Behälter umgedreht bzw. hängend befestigt wird. Der Einstechdorn ist vorzugsweise konisch ausgebildet, wobei die kanalförmige Ausnehmung des Anschlußstücks eine entsprechende Konizität aufweist. Zur besseren Abdichtung des Einstechdorns im Öffnungsbereich des rohrförmigen Abschnitts des Anschlußstücks ist dieses vorteilhafterweise an seinem freien Ende nach innen wulstartig erweitert.

In einer bevorzugten Ausführungsform der Erfindung weist die Schutzkappe sich in radialer Richtung erstreckende Flügel auf, so daß sich die Schutzkappe leicht greifen und das zum Abbrechen derselben erforderliche Drehmoment leicht aufbringen läßt. Die Schutzkappe ist so gestaltet, daß sie auch von ungeübten oder älteren Personen, beispielsweise im Home-Care-Bereich, leicht geöffnet werden kann.

Die Seitenwand der Schutzkappe ist vorteilhafterweise unterhalb der Ringbruchzone ringförmig verbreitert. Mit dem flanschartigen Unterteil kann die Schutzkappe mit einer ringförmigen Verbreiterung des Anschlußstücks verschweißt werden. Die Verbindung erfolgt in Abhängigkeit vom Material, dem Design und der Montageautomation beispielsweise durch Verkleben, Ultraschallschweißen, Heizelementschweißen oder Laserschweißen.

In einer bevorzugten Ausführungsform ist die Schutzkappe als Aufsteckkappe ausgebildet, so daß diese nach dem Durchtrennen der Sollbruchstelle wieder auf den rohrförmigen Abschnitt des Aufnahmestücks aufgesteckt werden kann. Da für die Verbindung von Schutzkappe und Anschlußstück kein Gewinde erforderlich ist, kann beim Spritzgießen die aufwendige Entspindelung und bei der Montage die Drehpositionierung sowie das anschließende Aufdrehen entfallen. Die Schutzkappe kann aber auch als Schraubkappe ausgebildet sein, so daß ein absolut dichter Wiederverschluß möglich ist.

In einer vorteilhaften Ausgestaltung ist das Ober- und Unterteil des Anschlußstücks nicht einstückig ausgebildet, sondern Ober- und Unterteil des Anschlußstücks weisen jeweils einen rohrförmigen Abschnitt auf, der mit einem Flansch versehen ist, so daß sich die Teile miteinander verschweißen lassen.

Bei einer zweiteiligen Ausführungsform ermöglicht das offene Unterteil, das in der Behälterwand eingesetzt und gegenüber der Behälterwand abgedichtet wird, den Behälter durch das Anschlußstück mit der medizinischen Flüssigkeit zu befüllen. Nach dem Befüllen des Behälters können Ober- und Unterteil an ihren Flanschen miteinander verschweißt werden, so daß der Behälter dicht verschlossen ist. Um das Befüllen zu erleichtern, kann der Kanal des Anschlußstückunterteils einen größeren Durchmesser haben als der Durchmesser des Kanals des Oberteils.

Wenn das Ober- und Unterteil einteilig ausgebildet sind, wird das Anschlußstück mit der Schutzkappe nach dem Befüllen des Beutels eingeschweißt. Der Behälter kann grundsätzlich aber auch durch das Anschlußstück befüllt und anschließend mit der Schutzkappe verschlossen werden.

Das Unterteil des Anschlußstücks bildet eine Halte- und Transportvorrichtung für den Behälter und schafft einen Zugang zu dem Behälterinneren, während das Oberteil mit der durchstechbaren Membran einen sterilisationsbeständigen und bakteriell sicheren Verschluß schafft (Originalitätsverschluß).

Die erfindungsgemäße Konnektoranordnung kann in flüssigkeitsgefüllten medizinischen Verpackungseinheiten unterschiedlichster Ausbildung Verwendung finden. Der Behälter, in den das Unterteil des Anschlußstücks eingesetzt wird, kann aus einem im wesentlichen starren Material bestehen oder kann als Folienbeutel ausgebildet sein. Wenn die Konnektoranordnung zum Befüllen und Verschließen eines Folienbeutels verwendet wird, kann das Unterteil aus einem relativ weichen Material bestehen, das sich mit der Folie leicht verschweißen läßt, ohne daß die Gefahr besteht, daß sich die Folie von dem Anschlußstück löst. Das Oberteil hingegen kann aus einem relativ harten Material bestehen, das eine ausreichende Festigkeit aufweist.

Das Unterteil des Anschlußstücks weist vorteilhafterweise sich in radiale Richtung erstreckende Flügel auf, die in einen Folienbeutel einschweißbar sind. Die Flügel des Anschlußstücks nehmen das beim Abbrechen der Schutzkappe auf den Folienbeutel aufgebrachte Drehmoment auf. Der Folienbeutel wird mit der einen Hand an den eingeschweißten Flügeln des Anschlußstücks gehalten, während die Schutzkappe mit der anderen Hand abgedreht wird.

Das Durchstoßelement ist vorteilhafterweise innerhalb des Anschlußstücks gegen Verdrehen gesichert. Hierzu können an der Innenwand des rohrförmigen Anschlußstücks mehrere umfangsmäßig verteilt angeordnete Rippen vorgesehen sein. Die Rippen begrenzen längslaufende Ausnehmungen, in die entsprechende längslaufende Vorsprünge eingreifen können, die an dem Einstechdorn vorgesehen sind. In dem rohrförmigen Abschnitt des Oberteils des Anschlußstücks kann aber auch eine längslaufende Führungsnut ausgebildet sein, in die ein an dem Einstechdorn vorgesehenes Führungsstück eingreift. Die Verdrehsicherung des Einstechdorns soll verhindern, daß die Membran beim Vorschieben des Dorns voll ausgestanzt wird und so unkontrolliert den Auslauf verstopft.

Die erfindungsgemäße Konnektoranordnung kann in vorteilhafter Weise zum Abfüllen und Verschließen eines Folienbeutels Verwendung finden, der eine medizinische Flüssigkeit, insbesondere eine Flüssigkeit für die enterale Ernährung enthält.

Im folgenden wird ein Ausführungsbeispiel der Erfindung unter Bezugnahme auf die Zeichnungen näher erläutert.

Es zeigen:
- Fig. 1: das Anschlußstück mit der Schutzkappe einer bevorzugten Ausführungsform der Konnektoranordnung in geschnittener Darstellung,
- Fig. 2: das Anschlußstück mit der Schutzkappe gemäß Fig. 1 in perspektivischer Darstellung,
- Fig. 3: das Anschlußstück und der in die kanalförmige Ausnehmung des Anschlußstücks eingeführte Einstechdorn zusammen mit der Überwurfmutter in geschnittener Darstellung,
- Fig. 4: die Konnektoranordnung gemäß Fig. 3 in perspektivischer Darstellung,
- Fig. 5: die Konnektoranordnung gemäß Fig. 3 mit aufgeschraubter Überwurfmutter in geschnittener Darstellung,
- Fig. 6: die Konnektoranordnung gemäß Fig. 5 in perspektivischer Darstellung und
- Fig. 7: einen mit dem Anschlußstück der Verbindungsanordnung verschlossenen Folienbeutel für die enterale Ernährung.

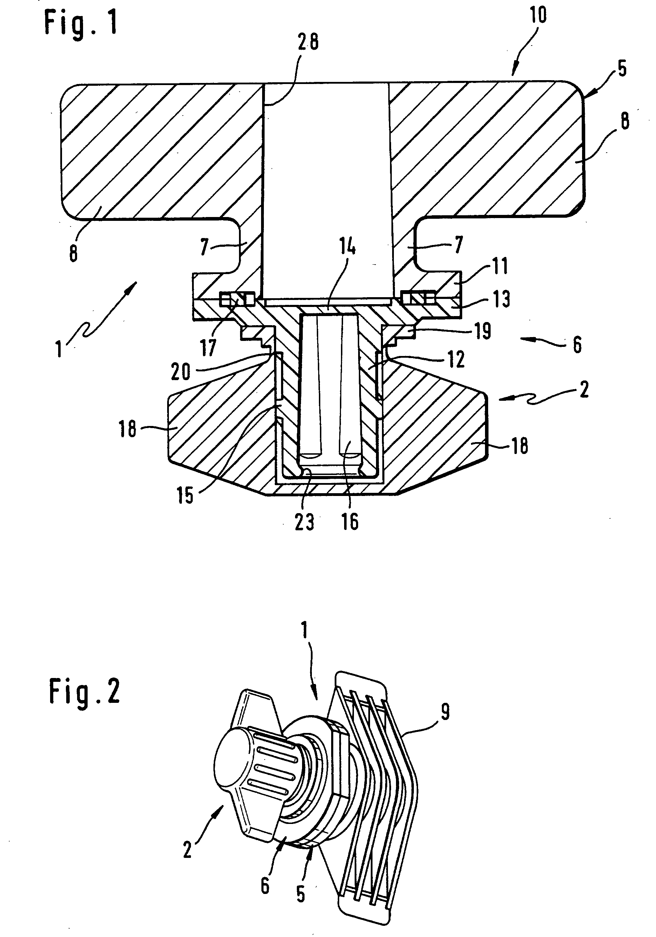

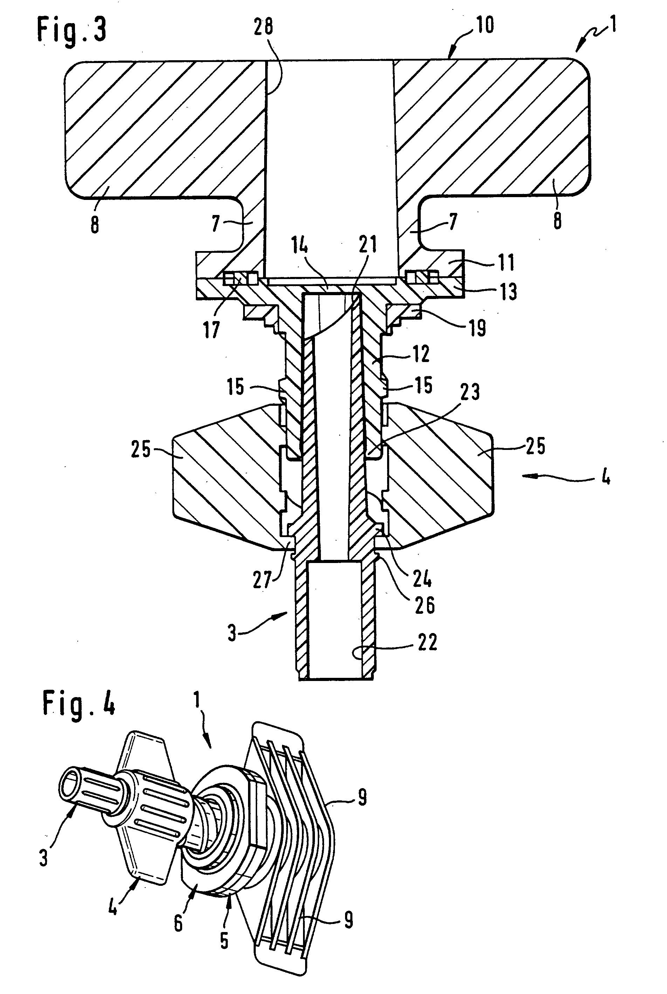

Die Konnektoranordnung umfaßt ein Anschlußstück 1, das in die Behälterwand einsetzbar und gegenüber der Behälterwand abdichtbar ist, eine abbrechbare Schutzkappe 2 (Fign. 1 und 2) und einen rohrförmigen Einstechdorn 3 mit einer Überwurfmutter 4 (Fign. 3 und 4).

Das Anschlußstück 1 besteht aus einem Unterteil 5 und einem Oberteil 6. Das Unterteil 5 des Anschlußstücks 1 weist einen rohrförmigen Abschnitt 7 auf, der mit zwei radial abstehenden flügelförmigen Ansätzen 8 versehen ist, die in einer Ebene liegen. Die flügelförmigen Ansätze 8 tragen jeweils vier parallele Rippen 9, die zu den Enden der flügelförmigen Ansätze 8 spitz zulaufen. Das nach Art eines Schiffchens ausgebildete Basisteil 10 des Anschlußstücks 1 kann in die bekannten Folienbeutel für medizinische Flüssigkeiten eingeschweißt werden. An seinem von dem Basisteil 10 vorstehenden Ende ist der rohrförmige Abschnitt 7 des Anschlußstückunterteils 5 mit einem Kreisringflansch 11 versehen.

Das Oberteil 6 des Anschlußstücks 1 weist ebenfalls einen rohrförmigen Abschnitt 12 mit einem Kreisringflansch 13 auf. An dem Ende, der dem Flansch 13 zugewandt ist, ist der rohrförmige Abschnitt 12 des Oberteils 6 mit einer Membran 14 verschlossen, die einstückiger Bestandteil des Oberteils ist. Die tiefliegende Membran 14, die aus dem gleichen Material wie der Port besteht, ist durch den rohrförmigen Abschnitt 12 des Anschlußstückoberteils 6 geschützt. Der rohrförmige Abschnitt 12 des Oberteils 6, der einen kleineren Außen- und Kanaldurchmesser als der rohrförmige Abschnitt 7 des Anschlußstückunterteils 5 aufweist, ist mit einem Außengewinde 15 versehen. An der Innenwand des rohrförmigen Abschnitts 12 sind mehrere umfangsmäßig verteilt angeordnete, längslaufende Rippen 16 vorgesehen.

Die Flansche 11, 13 des Ober- und Unterteils 5, 6 des Anschlußstücks 1, das als Spritzgußteil in großen Stückzahlen kostengünstig hergestellt werden kann, sind an ihren Berührungsflächen jeweils mit einem ringförmigen Steg 17 versehen und lassen sich mit einer geeigneten Schweißeinrichtung, die die Flansche von beiden Seiten erhitzt, auf einfache Weise unter Aufschmelzung der Stege miteinander verschweißen. Dabei liegt die Schweißnaht außerhalb der Füllstelle.

Zum bakteriellen Schutz der Füllstelle ist eine den rohrförmigen Abschnitt 12 des Anschlußstückoberteils übergreifende Schutzkappe 2 vorgesehen, die gleichsam als Originalitätsverschluß dient.

Die Schutzkappe 2 ist ein zylindrischer Körper, der mit zwei radial abstehenden Flügeln 18 versehen ist und an seinem unteren Rand einen Kreisringflansch 19 aufweist, der mit dem Kreisringflansch 13 des Anschlußstückoberteils beispielsweise durch Laserschweißen verbunden ist. Unmittelbar oberhalb des Kreisringflansches 19 ist an der Schutzkappe 2 eine Ringbruchzone 20 ausgebildet. Zum Öffnen der Einfüllstelle wird die Schutzkappe um ihre Längsachse gedreht, so daß die Wand der Schutzkappe an der ringförmigen Schwächungszone 20 bricht. Die abgebrochene Schutzkappe 2 kann bei Bedarf wieder auf das Anschlußstück 1 aufgesteckt werden.

Der rohrförmige Abschnitt 12 des Anschlußstückoberteils nimmt den Einstechdorn 3 auf (Fign. 3 und 4). Der rohrförmige Einstechdorn 3 (Spike) weist an einem Ende eine Spitze 21 auf und kann an seinem anderen Ende mit einem in den Figuren nicht dargestellten Kunststoffschlauch eines Schlauchleitungssystems verbunden werden, der in den durchgehenden Kanal 22 des Einstechdorns 3 eingeschoben wird. Die Länge des Einstechdorns 3 ist derart bemessen, daß er über einen Teil seiner Länge nach außen vorsteht, wenn er bis zu der Membran 14 vorgeschoben wird. Um eine sichere Abdichtung zu erzielen, ist der obere Rand 23 des rohrförmigen Abschnitts 12 des Anschlußstückoberteils nach innen ringförmig erweitert, so daß der rohrförmige Abschnitt beim Einschieben des Einstechdorns leicht aufgeweitet wird. Daher sollte das Anschlußstück aus einem leicht nachgiebigen Material bestehen.

An seinem nach außen vorstehenden Abschnitt weist der Einstechdorn 3 einen umlaufenden Rand 24 auf, an dem sich die auf den Dorn 3 aufgeschobene Überwurfmutter 4 abstützt. Die Überwurfmutter 4 ist an ihrer Außenseite gerippt und mit zwei radialen Flügeln 25 zum Festziehen versehen. Gegen Verlieren ist die Überwurfmutter 4 durch einen umlaufenden Steg 26 an dem schlauchseitigen Endstück des Einstechdorns 3 gesichert.

Beim Aufschrauben der Überwurfmutter 4 wird der Einstechdorn 3 in axialer Richtung auf die Membran 14 des Anschlußstücks 1 zubewegt, wobei diese durchstoßen wird. Da der Einstechdorn 3 in den Ausnehmungen geführt ist, die von den längslaufenden Rippen 16 in dem Oberteil 6 des Anschlußstücks 1 begrenzt werden, kann sich dieser nicht verdrehen. Dadurch wird verhindert, daß die Membran voll ausgestanzt wird und der Auslauf unkontrolliert verstopft.

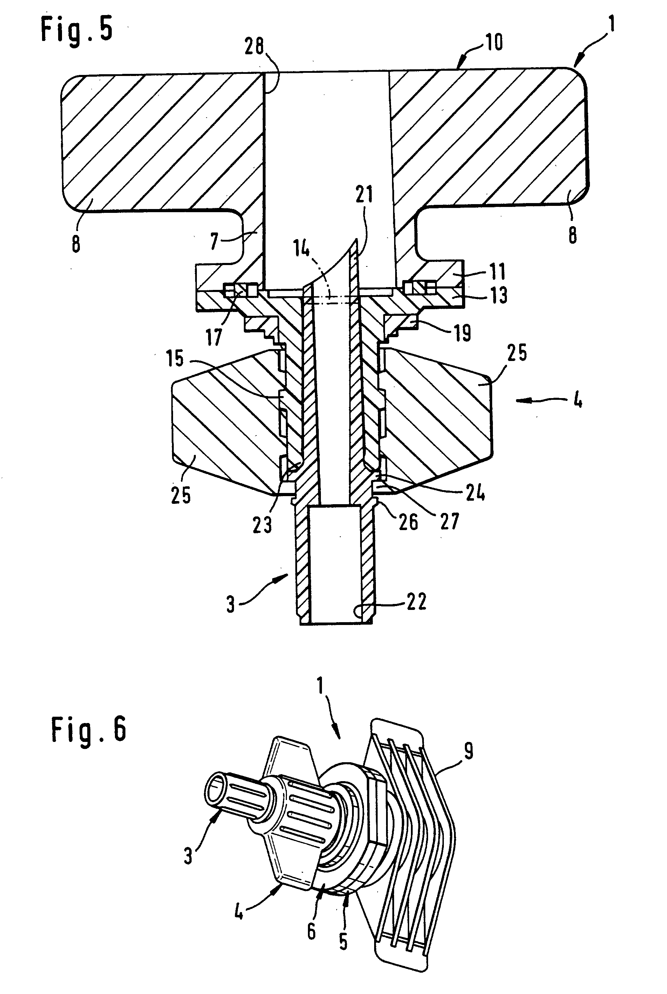

Die Fign. 5 und 6 zeigen die Konnektoranordnung bei durchstoßener Membran 14 und aufgeschraubter Überwurfmutter 4. Der umlaufende Ansatz 24 an der Außenseite des Einstechdorns 3 ist zwischen dem rohrförmigen Abschnitt 12 des Oberteils 6 und dem nach innen vorspringenden Rand 27 der Überwurfmutter 4 verklemmt. In dieser Position erstreckt sich die Spitze 21 des Einstechdorns 3 bis in die kanalförmige Ausnehmung 28 des Anschlußstückunterteils 5.

Sämtliche Teile der Verbindungsanordnung bestehen aus thermoplastischen Kunststoffen, vorzugsweise Polyolefine wie Polypropylen. Sie können im Spritzgußverfahren hergestellt werden.

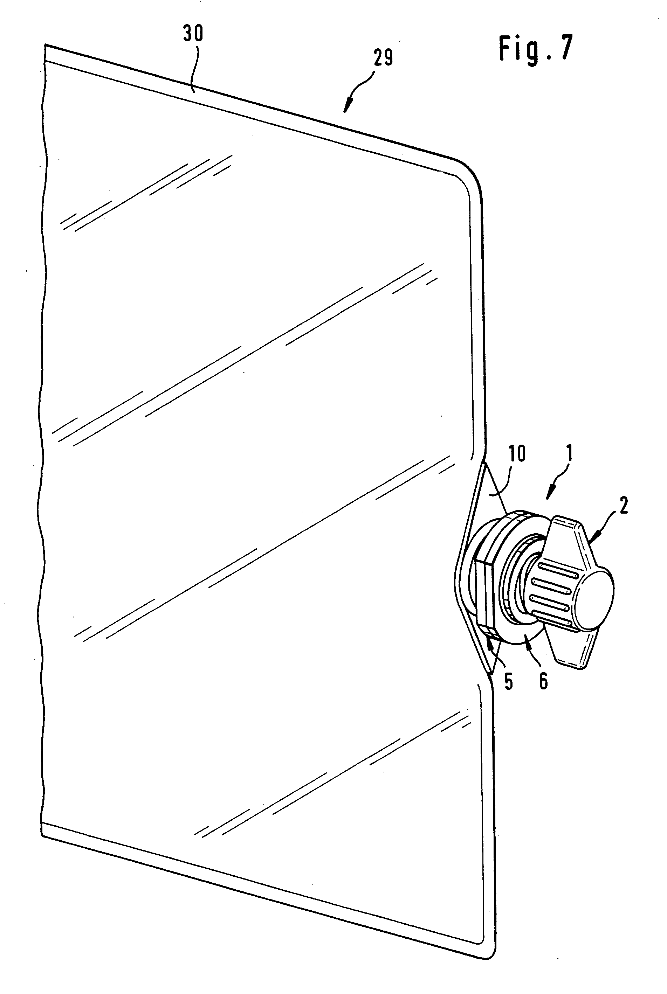

Fig. 7 zeigt einen Folienbeutel 29, der mit einer medizinischen Flüssigkeit für die enterale Ernährung gefüllt und mit dem Anschlußstück 1 der erfindungsgemäßen Konnektoranordnung verschlossen ist. Der Beutel 29 besteht aus zwei übereinanderliegenden an ihren Rändern 30 miteinander verschweißten Kunststoffolien, in die das Basisteil 10 des Anschlußstückunterteils 5 eingeschweißt ist. Das Oberteil 6 des Anschlußstücks 1 wird erst dann mit dem Unterteil 5 verschweißt, wenn der Behälter durch die kanalförmige Ausnehmung 28 des Unterteils mit der Flüssigkeit für die enterale Ernährung befüllt worden ist. An das Anschlußstück 1 kann nach dem Entfernen der Schutzkappe der mit einem Schlauchsystem verbundene Einstechdorn 3 eingeschoben und mittels der Überwurfmutter 4 festgezogen werden, wodurch eine Fluidverbindung zwischen dem Behälterinneren und dem Schlauchleitungssystem hergestellt wird.

## Patentansprüche

1. Sterile Konnektoranordnung zum Anschluß einer Schlauchleitung an einen eine medizinische Flüssigkeit enthaltenen Behälter mit
einem Anschlußstück (1), das mit einer kanalförmigen Ausnehmung (28) versehen ist, die von einer Membran (14) verschlossen ist, wobei das Anschlußstück (1) ein in die Behälterwand einsetzbares und gegenüber der Behälterwand abdichtbares Unterteil (5) und ein Oberteil (6) mit einem rohrförmigen Abschnitt (12) aufweist, und
einem in die kanalförmige Ausnehmung (28) des Anschlußstücks (1) einsetzbaren Einstechdorn (3), an dem die Schlauchleitung befestigbar ist,
**dadurch gekennzeichnet,**
**daß** der rohrförmige Abschnitt (12) des Anschlußstücks (1) mit einem Außengewinde (15) versehen ist,
**daß** eine den rohrförmigen Abschnitt (12) des Anschlußstücks (1) übergreifende Schutzkappe (2) vorgesehen ist, die mit ihrem unteren Rand mit dem Anschlußstück verbunden und im Bereich ihres unteren Randes mit einer Ringbruchzone (20) versehen ist, und
**daß** eine Überwurfmutter (4) vorgesehen ist, die nach dem Entfernen der Schutzkappe (2) auf den rohrförmigen Abschnitt (12) des Anschlußstücks (1) aufschraubbar ist, so daß der Einstechdorn (3) in dem Anschlußstück durch Drehen der Überwurfmutter in axialer Richtung verschiebbar ist.

2. Konnektoranordnung nach Anspruch 1, **dadurch gekennzeichnet, daß** die Schutzkappe (2) sich in radialer Richtung erstreckende Flügel (18) aufweist.

3. Konnektoranordnung nach Anspruch 1 oder 2, **dadurch gekennzeichnet, daß** die Seitenwand der Schutzkappe (2) unterhalb der Ringbruchzone (20) ringförmig verbreitert ist.

4. Konnektoranordnung nach einem der Ansprüche 1 bis 3, **dadurch gekennzeichnet, daß** der obere Rand des rohrförmigen Abschnitts (12) des Anschlußstücks (1) nach innen wulstartig erweitert ist.

5. Konnektoranordnung nach einem der Ansprüche 1 bis 4, **dadurch gekennzeichnet, daß** der Innendurchmesser der Schutzkappe (2) derart bemessen ist, daß die Schutzkappe nach dem Durchtrennen der Ringbruchzone (20) klemmend auf dem rohrförmigen Abschnitt (12) des Anschlußstücks (1) fixiert ist.

6. Konnektoranordnung nach einem der Ansprüche 1 bis 5, **dadurch gekennzeichnet, daß** die Überwurfmutter (4) an dem Einstechdorn (3) unverlierbar befestigt ist.

7. Konnektoranordnung nach einem der Ansprüche 1 bis 6, **dadurch gekennzeichnet, daß** das Unterteil (5) des Anschlußstücks (1) einen rohrförmigen Abschnitt (7) aufweist, wobei der rohrförmige Abschnitt des Unterteils und der rohrförmige Abschnitt (12) des Oberteils (6) jeweils mit einem Flansch (11, 13) versehen und an ihren Flanschen miteinander verschweißt sind.

8. Konnektoranordnung nach einem der Ansprüche 1 bis 7, **dadurch gekennzeichnet, daß** das Unterteil (5) des Anschlußstücks (1) sich in radialer Richtung erstreckende Flügel (8) aufweist, die in einen Folienbeutel einschweißbar sind.

9. Konnektoranordnung nach einem der Ansprüche 1 bis 8, **dadurch gekennzeichnet, daß** der Einstechdorn (3) und/oder das Anschlußstück (1) derart ausgebildet sind, daß der Einstechdorn (3) in der kanalförmigen Ausnehmung des Anschlußstücks (1) gegen Verdrehen gesichert ist.

10. Konnektoranordnung nach einem der Ansprüche 1 bis 9, **dadurch gekennzeichnet, daß** die Teile der Konnektoranordnung aus Polyolefinen, insbesondere Polypropylen, bestehen.

11. Behälter mit einer sterilen Konnektoranordnung nach einem der Ansprüche 1 bis 10, **dadurch gekennzeichnet, daß** das Unterteil (5) des Anschlußstücks (1) in die Behälterwand eingesetzt und gegenüber der Behälterwand abgedichtet ist und der Behälter mit einer medizinischen Flüssigkeit gefüllt ist.

12. Behälter nach Anspruch 11, **dadurch gekennzeichnet, daß** der Behälter ein Folienbeutel ist.

13. Behälter nach Anspruch 12 oder 13, **dadurch gekennzeichnet, daß** die medizinische Flüssigkeit eine Flüssigkeit für die enterale Ernährung ist.

## Claims

1. A sterile connector arrangement for connecting a tube to a container containing medical fluid, with
a connecting piece (1) provided with a channel-like cut-out (28) which is occluded by a diaphragm (14), the connecting piece (1) comprising a bottom part (5) which can be inserted into the container wall and sealed in respect of the container wall and an upper part (6) with a tubular portion (12), and
an insertion piece (3) adapted to be fitted into the channel-like cut-out (28) in the connecting piece (1) and on which the tube can be fitted,
**characterised in that**
the tubular portion (12) of the connecting piece (1) has an external screw thread (15),
and **in that** a protective cap (2) is provided to engage over the tubular portion (12) of the connecting piece (1), its bottom edge being connected to the connecting piece and being provided in the region of its bottom edge with an annular fracture zone (20), and
**in that** a cap nut (4) is provided which, after removal of the protective cap (2), can be screwed onto the tubular portion (12) of the connecting piece (1) so that the insertion mandrel (3) in the connecting piece can be displaced in an axial direction by rotating the cap nut.

2. A connecting arrangement according to claim 1, **characterised in that** the protective cap (2) has wings (18) extending in a radial direction.

3. A connecting arrangement according to claim 1 or 2, **characterised in that** the side wall of the protective cap (2) is widened out annularly below the annular fracture zone (20).

4. A connecting arrangement according to one of claims 1 to 3, **characterised in that** the upper edge of the tubular portion (12) of the connecting piece (1) is inwardly widened in a bead-like fashion.

5. A connecting arrangement according to one of claims 1 to 4, **characterised in that** the inside diameter of the protective cap (2) is so dimensioned that, once the annular fracture zone (20) has been broken through, the protective cap is fixed in clamping manner on the tubular portion (12) of the connecting piece (1).

6. A connecting arrangement according to one of claims 1 to 5, **characterised in that** the cap nut (4) is fixed in captive manner on the insertion mandrel (3).

7. A connecting arrangement according to one of claims 1 to 6, **characterised in that** the bottom part (5) of the connecting piece (1) comprises a tubular portion (7), the tubular portion of the bottom part and the tubular portion (12) of the upper part (6) being respectively provided with a flange (11, 13) and are welded to each other at their flanges.

8. A connecting arrangement according to one of claims 1 to 7, **characterised in that** the bottom part (5) of the connecting piece (1) has, extending in a radial direction, wings (8) which can be welded into a film bag.

9. A connecting arrangement according to one of claims 1 to 8, **characterised in that** the insertion mandrel (3) and/or the connecting piece (1) are so constructed that the insertion mandrel (3) in the channel-like cut-out in the connecting piece (1) is secured against rotation.

10. A connecting arrangement according to one of claims 1 to 9, **characterised in that** the parts of the connecting arrangement consist of polyolefins, in particular of polypropylene.

11. A container with a sterile connecting arrangement according to one of the claims 1 to 10, **characterised in that** the bottom part 5 of the connecting piece 1 is inserted into the container wall and is sealed in respect of the container wall, and **in that** the container is filled with a medicinal fluid.

12. A container according to claim 11, **characterised in that** the container is a film bag.

13. A container according to claim 12 or 13, **characterised in that** the medicinal fluid is a fluid for enteral nourishment.

## Revendications

1. Ensemble de connexion stérile destiné au branchement d'un tuyau à un récipient contenant une préparation médicamenteuse liquide, comprenant une pièce de raccord (1) qui est munie d'une cavité en forme de canal (28), qui est fermée par une membrane (14), la pièce de raccord (1) étant formée par une partie inférieure (5) susceptible d'être insérée dans la paroi du récipient et d'être rendue étanche par rapport à la paroi du récipient, et par une partie supérieure (6) munie d'une partie tubulaire (12), un poinçon (3) susceptible d'être introduit dans la cavité en forme de canal (28) de la pièce de raccord (1) et pouvant être fixé à un tuyau, **caractérisé en ce que** la partie tubulaire (12) de la pièce de raccord (1) est munie d'un filetage extérieur (15), **en ce qu'**il est prévu un capuchon de protection (2) posé sur la partie tubulaire (12) de la pièce de raccord (1), lequel capuchon est assemblé avec son bord inférieur contre la pièce de raccord et est muni d'une zone de rupture circulaire (20) dans la région de son bord inférieur, et **en ce qu'**il est prévu un écrou à pattes (4) qui, une fois que le capuchon de protection (2) a été retiré, peut être vissé sur la partie tubulaire (12) de la pièce de raccord (1), de telle sorte que le poinçon (3) peut coulisser dans la pièce de raccord, sous l'effet de la rotation de l'écrou à pattes, dans le sens axial.

2. Ensemble de connexion selon la revendication 1, **caractérisé en ce que** le capuchon de protection (2) comporte des ailettes (18) orientées dans le sens radial.

3. Ensemble de connexion selon la revendication 1 ou 2, **caractérisé en ce que** la paroi latérale du capuchon de protection (2) s'élargit de manière circulaire en dessous de la zone de rupture circulaire (20).

4. Ensemble de connexion selon l'une quelconque des revendications 1 à 3, **caractérisé en ce que** le bord supérieur de la partie tubulaire (12) de la pièce de raccord (1) s'élargit vers l'intérieur en formant un bourrelet.

5. Ensemble de connexion selon l'une quelconque des revendications 1 à 4, **caractérisé en ce que** le diamètre intérieur du capuchon de protection (2) est choisi de telle sorte que le capuchon de protection, après la cassure de la zone de rupture circulaire (20), est bloqué par serrage sur la partie tubulaire (12) de la pièce de raccord (1).

6. Ensemble de connexion selon l'une quelconque des revendications 1 à 5, **caractérisé en ce que** l'écrou à pattes (4) est fixé de manière imperdable contre le poinçon (3).

7. Ensemble de connexion selon l'une quelconque des revendications 1 à 6, **caractérisé en ce que** la partie inférieure (5) de la pièce de raccord (1) comporte une partie tubulaire (7), la partie tubulaire de la partie inférieure et la partie tubulaire (12) de la partie supérieure (6) étant munies chacune d'une collerette (11, 13) et étant soudées l'une contre l'autre au niveau de leurs collerettes.

8. Ensemble de connexion selon l'une quelconque des revendications 1 à 7, **caractérisé en ce que** la partie inférieure (5) de la pièce de raccord (1) comporte des ailettes (8) orientées dans le sens radial, qui peuvent être soudées dans une poche souple en plastique.

9. Ensemble de connexion selon l'une quelconque des revendications 1 à 8, **caractérisé en ce que** le poinçon (3) et/ou la pièce de raccord (1) sont conçus de telle sorte que le poinçon (3) est positionné dans la cavité en forme de canal de la pièce de raccord (1) de manière à ne pas pouvoir tourner.

10. Ensemble de connexion selon l'une quelconque des revendications 1 à 9, **caractérisé en ce que** les pièces du ensemble de connexion sont réalisées dans des polyoléfines, tels que le polypropylène.

11. Récipient comprenant un ensemble de connexion selon l'une quelconque des revendications 1 à 10, **caractérisé en ce que** la partie inférieure (5) de la pièce de raccord (1) est insérée dans la paroi du récipient et est rendue étanche par rapport à la paroi du récipient, et le récipient contient une préparation médicamenteuse liquide.

12. Récipient selon la revendication 11, **caractérisé en ce que** le récipient est une poche souple.

13. Récipient selon la revendication 11 ou 12, **caractérisé en ce que** la préparation médicamenteuse est un liquide pour alimentation par voie entérale.
